# EUROPEAN PATENT SPECIFICATION

(11) **EP 0 567 102 B1**
(45) Date of publication and mention of the grant of the patent: **19.07.2000**
(21) Application number: 93106499.2
(22) Date of filing: 21.04.1993
(51) Int. Cl.: C12N 1/20, C02F 3/34, A62D 3/00, C12P 17/04

(54) **Method of biologically decomposing phenol or furan compounds by microorganisms**
Verfahren zur biologischen Zersetzung von Phenol- oder Furanverbindungen mit Mikroorganismen
Procédé de décomposition biologique de composés phénolique ou furanique avec des microorganismes

(30) Priority: 22.04.1992 JP 10318092; 31.07.1992 JP 20491392
(43) Date of publication of application: 27.10.1993
(73) Proprietor: CANON KABUSHIKI KAISHA, Tokyo (JP)
(72) Inventor: Kato, Kinya, c/o CANON KABUSHIKI KAISHA, Ohta-ku, Tokyo (JP); Sakuranaga, Masanori, c/o CANON KABUSHIKI KAISHA, Ohta-ku, Tokyo (JP); Kozaki, Shinya, c/o CANON KABUSHIKI KAISHA, Ohta-ku, Tokyo (JP)
(74) Representative: Bühling, Gerhard, Dipl.-Chem.

(56) References cited:
- EP-A- 0 090 652
- EP-A- 0 095 049
- WO-A-92/19738
- DE-A- 2 752 380
- DATABASE WPI Week 9151, Derwent Publications Ltd., London, GB; AN 91-373416 & JP-A-3 251 178 (SUEMITSU) 8 November 1991
- APPLIED AND ENVIROMENTAL MICROBIOLOGY vol. 57, no. 7 , July 1991 , WASHINGTON D.C.,USA pages 1935 - 1941 SHIELDS ET AL 'MUTANTS OF PSEUDOMONAS CEPACIA G4 DEFECTIVE IN CATABOLISM OF AROMATIC COMPOUNDS AND TRICHLOROETHYLENE'

## Description

### BACKGROUND OF THE INVENTION

### Field of the Invention

The present invention relates to a method of decomposing phenolic compounds or furan compounds using microorganisms which are derived from the intestine of a termite, a method of obtaining microorganisms having the ability to decompose phenolic compounds or furan compounds, and a novel strain derived from the intestine of a termite having the ability to decompose phenolic compounds or furan compounds. The present invention also relates to a method of producing 2-furan carboxylic acid from furfural employing the intestinal microorganisms of a termite.

### Description of the Related Art

In recent years, toxic aromatic compounds which cannot be easily decomposed under natural conditions have been detected by various environmental surveys, and the environmental contamination caused by these compounds has been publicized. It has also been feared that these compounds adversely affect human bodies. In order to prevent the contamination by non-decomposable chemical substances, it is urgent to develop a technique of preventing the escape of these chemical substances into the environment. For example, there is a great demand for establishing a technique of effectively removing non-decomposable toxic substances from waste water.

Examples of such non-decomposable substances include phenolic compounds such as phenol and cresol; furan compounds such as furfural, tetrahydrofuran, furfuryl alcohol, and cumaran.

Although phenol found in various waste liquids can be decomposed by a chemical decomposition method using light, heat, ozone or the like, microbial decomposition attracts attention from the viewpoints of treatment cost, and operation. Examples of microorganisms known as having the ability to decompose phenol include bacteria belonging to Pseudomonas, Nocardia, Bacillus, Acinetobacter, Aureobasidium; fungi belonging to Fusarium and the like; yeasts belonging to Triccosporon, Candida and the like. Typical examples of bacteria belonging to the Pseudomonas include Pseudomonas nutida, and Pseudomonas paucimobilis.

EP-A-0 095 049 discloses a treatment method for decomposing organic compounds including phenolic compounds present in waste effluents by treating the organic compounds with activated sludge comprising *Pseudomonas cepacia* var. *niagarous*.

Cresol is found in waste liquids of coal gasification factories, groundwater contaminated with gasoline, waste liquids of petroleum refineries and the like. The purification of such waste liquids by decomposing cresol becomes a critical problem from the viewpoint of environmental protection. Although cresol can also be decomposed by a chemical decomposition method using light, heat, ozone or the like, microbial decomposition is appreciated from the viewpoints of the treatment cost and operation. However, there is substantially no isolated microorganism having the ability to decompose cresol, and only few bacteria belonging to Pseudomonas such as Pseudomonas QT31 strain (C. Masque et al., Biotechnology Letters, Vol. 9, No. 9, 655 - 660, 1987) and the like are reported as cresol resistant strains.

There are only few reports on isolated microorganisms having the ability to decompose furfural or tetrahydrofuran. Such reports include a report on decomposition of furfural by culturing bread yeasts (S. Morimoto et al., J. Ferment. Technol., 45, 442, 1967), and an old-Soviet patent (Patent Japan 44-20389) on conversion of furfural to 2-furan carboxylic acid by Acetobacter, Achromobacter, Brevibacterium, Flavobacterium, Micrococcus or the like (refer to "Conversion of Organic Compound by Microorganism", by G. K. Skryabin and L. A. Golovleva, translated by Saburo Fukui, Gakkai Shupan Center, 287). An experiment performed under anaerobic conditions is described in Brune, G., Schovert h, S. M., and Sahm , H. (1982); Process Biochem., 17, 20. There is substantially no example of isolated microorganisms having the ability to decompose tetrahydrofuran or cumaran.

There is, thus, a great demand for practical reasons for obtaining microorganisms having the ability to decompose such non-decomposable furan compounds. As is common to aldehydes, furfural among these furan compounds has high toxicity. Thus, it is important to obtain microorganisms having resistance to the toxicity of furfural. In addition, in treatment of a compound having a cumaran ring as a basic skeleton thereof, microorganisms having the ability to decompose the cumaran ring is useful and is, therefor in great demand.

On the other hand, 2-furan carboxylic acid among the furan compounds has lower toxicity than that of furfural and is easily assimilated by microorganisms. It is to be expected that the conversion of furfural available at low cost and furan compounds mixed in various waste liquids to 2-furan carboxylic acid promotes the utilization of furan compounds in the fermentation industry.

In particular, the oxidative conversion of furfural to 2-furan carboxylic acid is a very significant means for decreasing the toxicity of furfural and introducing it as a raw material into the fermentation industry. Known methods of converting furfural to 2-furan carboxylic acid include various methods using chemical reactions. However, any one of the methods requires violent reaction, and produces high concentrations of various toxic substances in the final reaction solutions which inhibit the growth of microorganisms. When the 2-furan carboxylic acid obtained by the chemical method is used as a raw material for fermentation, it requires a troublesome operation of separating the toxic substances from the final reaction solution. From this viewpoint, attention is paid to a method of producing 2-furan carboxylic acid by using microorganisms to obtain a target substance under very mild conditions.

However, as described above, there are only a few reports on isolated microorganisms used for converting furfural to 2-furan carboxylic acid. There is, thus, a demand to obtain practical useful microorganisms.

Furfural can easily be obtained, for example, by steam distillation of a plant containing pentosan or treatment thereof with a mineral acid, and is industrially produced from hulls of oats or wheat straws. Of the furan compounds, furfural is available at the lowest cost and is widely used as a selective purification solvent for lubricating oil, a vulcanization accelerator, a dye penetrating agent and the like.

Tetrahydrofuran is useful as an organic solvent having excellent dissolving ability and is widely used as a solvent component for surface coating, an adhesive, a paint release agent and the like. Frufuryl alcohol is used as a solvent or a dispersant for dyes, phenolic resins, and a lubricant. A compound having a cumaran (2,3-dihydrobenzofuran) ring is widely used for production of dyes and pigments, as an organic solvent. The compound is also found as a component of wood.

The above compounds are thus mixed in waste liquids of various chemical factories, and the presence of tetrahydrofuran presents the danger of contaminating groundwater and the like because of water solubility. Accordingly, it is important from the viewpoint of environmental protection to decompose these compounds.

Although the non-decomposable compounds can be decomposed by a method using light, heat, ozone or the like, a biological decomposition method employing a microorganism is appreciated from the viewpoints of the cost and operation properties.

There are known microorganisms having the ability to decompose phenolic compounds which include the above strains. However, satisfactory strains which satisfy practical conditions and have a sufficient decomposing ability when used for decomposing a phenolic compound using microorganisms are not found in the currently known strains. It is thus necessary to obtain a strain which satisfies the characteristics required for practical use.

In addition, only few microorganisms are known as microorganisms having the ability to decompose furan compounds which are not easily decomposed under natural conditions, and strains which satisfy practical conditions and have the sufficient decomposing ability are not currently known. Therefor, it is necessary to obtain a strain which satisfies the characteristics required for practical use.

Similarly, satisfactory microorganisms which are employed for producing 2-furan carboxylic acid from a furan compound are not found, and it is necessary to obtain a strain which satisfies the characteristics required for practical use.

The characteristics required for practical use are as follows: (1) A strain must have the ability to decompose a phenolic compound or a furan compound or the ability to convert a furfural compound to 2-furan carboxylic acid; (2) A broader range of growth conditions than those of known strains; (3) The applicability can be increased, or a strain can be utilized in various forms.

For example, microorganisms used for treating a waste water containing a phenolic compound or a furan compound are required not to be damaged easily in the waste water, necessitating microorganisms that can be grown under poor conditions, as in a waste water. Microorganisms having resistance to many antibiotics and the ability to utilize various kinds of sugar have the greater possibility to grow well under poor environmental conditions. It is thus important to obtain microorganisms having resistance to various chemicals, as well as high utilization of sugar.

At an actual contamination site, composite contamination is frequently produced by several chemical substances rather than a single chemical substance. A treatment method using different kinds of bacteria for decomposing the respective chemical substances causes a problem with respect to differences in the growth conditions of the bacteria used and requires more complicated control. There is thus a demand for microorganisms of a single strain having the ability to several chemical substances.

Accordingly, there is a great demand for a strain having the ability to decompose a phenolic compound or a furan compound that is more practical and useful than conventional known strains.

### SUMMARY OF THE INVENTION

Accordingly, an object of the present invention is to provide a practical method of decomposing a phenolic compound using microorganisms.

Another object of the present invention is to provide a method of obtaining useful microorganisms for decomposing a phenolic compound.

Still another object of the present invention is to provide a novel strain having the ability to decompose a phenolic compound as well as characteristics more practical and useful than conventional known strains.

A further object of the present invention is to provide a practical method of biologically decomposing a furan compound using appropriate microorganisms.

A still further object of the present invention is to provide a method of obtaining microorganisms useful for decomposing a furan compound.

A further object of the present invention is to provide a practical method of producing 2-furan carboxylic acid using appropriate microorganisms.

In order to achieve the object, the present invention provides a method of biologically decomposing a phenolic compound comprising the step of decomposing a phenolic compound by bringing an aqueous solution containing the phenolic compound into contact with the intestinal microorganisms of termites.

The present invention further provides a method of obtaining microorganisms having the ability to decompose a phenolic compound comprising the steps of culturing the microorganisms isolated from the intestines of termites in a medium containing a phenolic compound as a single carbon source and recovering the grown microorganisms.

The present invention still further provides a method of biologically decomposing a furan compound comprising the step of decomposing a furan compound in the presence of the intestinal microorganisms of termites having the ability to decompose the furan compound.

The present invention further provides a method of obtaining microorganisms having the ability to decompose a furan compound comprising the steps of culturing the microorganisms isolated from the intestines of termites in a medium containing a furan compound as a single carbon source, and recovering the grown microorganisms.

The present invention further provides an isolated strain of Pseudomonas cepacia KK01 (Ferm BP4235) derived from the intestine of a termite and having the ability to decompose a phenolic compound or a furan compound.

The present invention further provides a method of producing 2-furan carboxylic acid comprising the step of oxidizing furfural by means of the intestinal microorganisms of termites to obtain 2-furan carboxylic acid.

### BRIEF DESCRIPTION OF THE DRAWINGS

Fig. 1 is a graph showing daily changes of the residual rate of phenol in a culture medium measured in Example 1;
Fig. 2 is a graph showing a difference in the ultraviolet absorption spectra of a culture solution measured before and after culture in Example 2;
Fig. 3 is a graph showing a difference in the ultraviolet absorption spectra of a culture solution measured before and after culture in Example 3;

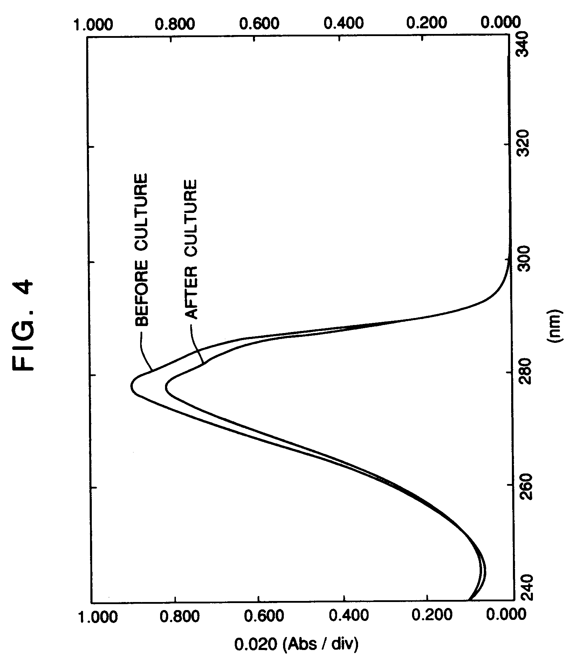

Fig. 4 is a graph showing a difference in the ultraviolet absorption spectra of a culture solution measured before and after culture in Example 4;
Fig. 5 is a graph showing daily changes of the residual rate of phenol in a culture medium measured in Example 5;
Fig. 6 is a graph showing a difference in the ultraviolet absorption spectra of a culture solution measured before and after culture in Example 9;
Fig. 7 is a graph showing a difference in the ultraviolet absorption spectra of a culture solution measured before and after culture in Example 9;
Fig. 8 is a graph showing a difference in the ultraviolet absorption spectra of a culture solution measured before and after culture in Example 9;
Fig. 9 is a graph showing daily changes in the residual rate of a phenolic compound in a waste liquid measured in Example 10;
Fig. 10 is a graph showing showing a difference in the ultraviolet absorption spectra of a culture solution measured before and after culture in Example 11;
Fig. 11 is a graph showing showing a difference in the ultraviolet absorption spectra of a culture solution measured before and after culture in Example 13;
Fig. 12 is a graph showing showing a difference in the ultraviolet absorption spectra of a culture solution measured before and after culture in Example 15;
Fig. 13 is a graph showing showing a difference in the ultraviolet absorption spectra of a culture solution measured before and after culture in Example 17;
Fig. 14 is a graph showing showing a difference in the ultraviolet absorption spectra of a culture solution measured before and after culture in Example 19;
Fig. 15 is a graph showing showing a difference in the ultraviolet absorption spectra of a culture solution measured before and after culture in Example 20;
Fig. 16 is a graph showing showing a difference in the ultraviolet absorption spectra of a culture solution measured before and after culture in Example 21;
Fig. 17 is a graph showing showing a difference in the ultraviolet absorption spectra of a culture solution measured before and after culture in Example 22; and
Fig. 18 is a graph in which a change with time of the amount of a furan compound in a culture medium measured in Example 23 is shown by a residual rate relative to the amount before culture which is considered as 100 %.

### DESCRIPTION OF THE PREFERRED EMBODIMENTS

The microorganisms used in a decomposition method of the present invention and derived from the intestines of termites can be obtained by, for example, washing the surfaces of termites so as to sterilize the surfaces, extracting the intestines and grinding them in an appropriate solution, adding a part of the resulting mixture containing the ground intestines to a culture medium containing as a single carbon source a phenolic compound or a furan compound to be decomposed, and isolating the grown strain. Although various kinds of termites can be used, termites belonging to the genuses Nasutiterminae such as Nasutitermes takasagoensis, Nasutitermes ephratae, Nasutitermes exitiosus, Nasutitermes nigriceps and the like are preferable. Nasutitermes takasagoensis is particularly preferable.

A medium containing as a single carbon source a phenolic compound or a furan compound, and if required, various nitrogen sources, inorganic salts, growth factors and the like, can be used as a medium for screening microorganisms having the ability to decompose the phenolic compound or furan compound. For example, in the case of the bacteria belonging to Pseudomonas, yeast extract and peptone can be used singly or in combination thereof as a nitrogen source, and potassium hydrogen primary phosphate, ammonium chloride or the like can be used as an inorganic salt. The concentration of the phenolic compound can be appropriately selected, for example, to be set in the range of 0.02 to 0.07 %. The concentration of the furan compound can be appropriately selected, for example, to be set in the range of 0.01 to 0.05 %.

The thus-isolated microorganisms can be cultured under conditions appropriately selected which are suitable for the microorganisms.

The phenolic compound or furan compound can be decomposed by using the isolated microorganisms. A mixture of at least two microorganisms having the ability to decompose the phenolic compound or furan compound may be used for decomposing the compound. When a mixture is used, a mixture having a known microorganism composition or having an unknown composition and exhibiting the ability to decompose the phenolic compound or furan compound can be used. Even when the screening medium contains various kinds of microorganisms, the medium can be used as a mixture system without isolation of the respective microorganisms. The Pseudomonas cepacia KK01 strain or the like can be used as an isolated strain. A mutant obtained by naturally or artificially modifying the intestinal microorganisms of termites and having the ability to decompose the phenolic compound or the furan compound can also be used in the present invention.

The KK01 stain has resistance to various kinds of antibiotics, and permits various kinds of sugar to be utilized as a carbon source, as described in the examples below. The strain also has the ability to decompose phenol, the ability to decompose cresol and the ability to decompose tetrahydrofuran, furfuryl alcohol and cumaran. The KK01 strain is thus practically useful for biologically decomposing phenolic and furan compounds.

In the present invention, a phenolic compound or a furan compound contained in a substance such as waste water to be treated can be decomposed by bringing the phenolic compound or furan compound into contact with the intestinal microorganisms of termites. The contact between the microorganisms and the substance to be treated can be performed by a method of culturing the microorganisms in an aqueous solution containing the phenolic compound or furan compound to be decomposed, or by a method of adding the aqueous solution to a culture system of the microorganisms. The methods can be performed in one of various systems such as a batch system, a semi-continuous system, a continuous system and the like. The microorganisms can be used in an unbound state or state wherein they are bound to an appropriate carrier. The substance such as waste water to be treated may be subjected to various pretreatments according to need. For example, pretreatment may be preformed for controlling the concentration of the phenolic compound or the furan compound and the pH of the liquid, or supplying various nutritive substances such as the yeast extract and the like. The concentration of the phenolic compound within the decomposition region may be adjusted to about 0.2 % or less in the presence of other nutritive substances such as yeast extract and the like. For example, the concentration of the furan compound within the decomposition region may be adjusted to about 0.05 % in the presence of other nutritive substances such as the yeast extract and the like.

The 2-furan carboxylic acid producing method of the present invention comprises the step of obtaining 2-furan carboxylic acid from furfural or furfuryl alcohol by means of the intestinal microorganism of termites.

The microorganisms used for producing 2-furan carboxylic acid can be obtained by selecting microorganisms which produce 2-furan carboxylic acid from the microorganisms obtained by the screening and having the ability to decompose furfural or furfuryl alcohol.

In the present invention, 2-furan carboxylic acid can be produced by bringing furfural into contact with the intestinal microorganisms of termites. The contact of a raw material compound with the microorganisms can be performed by a method of culturing the microorganisms in an aqueous solution containing the raw material compound, or a method of adding the aqueous solution to a culture system of the microorganisms. These methods can be performed in one of various systems such as a batch system, a semi-continuous system, a continuous system and the like. The microorganisms can be used in an unbound state or a state wherein they are bound to an appropriate carrier. The concentration of the raw material compound, i.e., furfural, pH, and the concentrations of various nutritive substances can be appropriately selected in accordance with the microorganisms used. For example, the concentration of the raw material compound may be adjusted to about 0.05 % in the presence of other nutritive substances such as yeast extract. The culture is preferably performed under aerobic conditions at about 30°C. When waste water containing the raw material compound is used, pretreatment may be made for adjusting the concentration of the raw material compound and the pH of the liquid, or supplying various nutritive substances.

### EXAMPLES

The present invention is described in further detail below with reference to examples. The M9 medium used in each of the examples has the following composition:

| M9 medium composition (per liter) | |
|---|---|
| NaHPO₄ | 6.2 g |
| KH₂PO₄ | 3.0 g |
| NaCl | 0.5 g |
| NH₄Cl | 1.0 g |
| (pH 7.0) | |

### Example 1 (Screening by phenol)

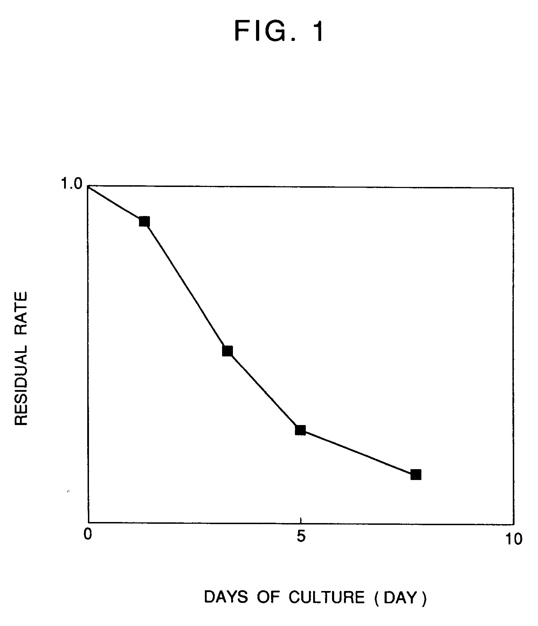

Ten worker termites Nasutitermes takasagoensis were placed on a plate, and ethyl alcohol (95 %) was poured into the plate so as to sterilize the surfaces of the termites. The ethyl alcohol was removed by washing the termites twice in the M9 medium containing 0.05 % phenol. After washing, the intestine was picked out from each termite with a pair of tweezers, and was then ground in the M9 medium containing 0.05 % phenol to obtain a liquid mixture containing the ground intestines. A portion of the resulting mixture was inoculated into the M9 medium containing 0.05 % phenol and 0.05 % yeast extract, followed by culture under aerobic conditions at 30°C. The daily change in the amount of phenol contained in the medium was measured. The amount of the phenol contained in the medium was measured by the method below. The medium was sampled, and the sample obtained was passed through a filter of 0.2 µm. The concentration of phenol in the filtrate obtained was determined by measuring light absorption at about 270 nm using a spectrophotometer. The phenol concentration was indicated by a residual rate of phenol. The results obtained are shown in Fig. 1. The results shown in Fig. 1 reveals that microorganisms which assimilate phenol are present in the intestine of a termite.

### Example 2 (Screening by o-cresol)

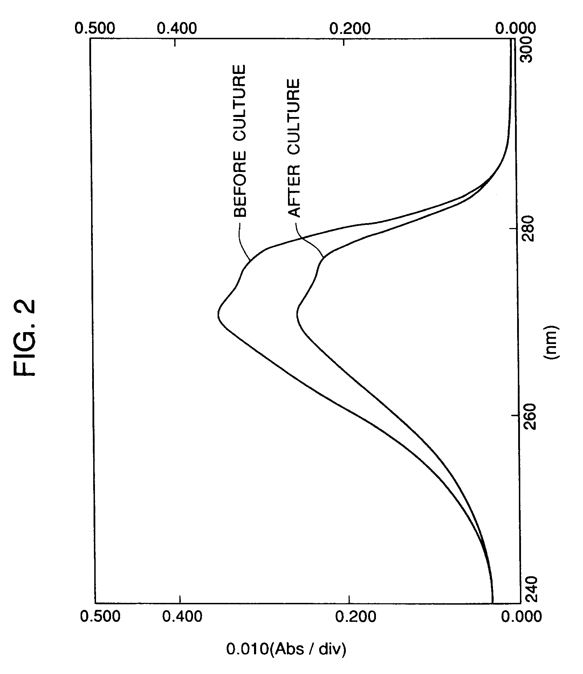

Ten worker termites Nasutitermes takasagoensis were placed on a plate, and ethyl alcohol (95 %) was poured into the plate so as to sterilize the surfaces of the termites. The ethyl alcohol was removed by washing the termites twice in the M9 medium containing 0.02 % o-cresol. After washing, the intestine was picked out from each termite with a pair of tweezers, and was then ground in the M9 medium containing 0.02 % o-cresol to obtain a liquid mixture containing the ground intestines. A portion of the resulting mixture was inoculated into the M9 medium containing 0.02 % o-cresol and 0.05 % yeast extract, followed by culture under aerobic conditions at 30°C for 10 days. A difference in the amounts of o-cresol contained in the M9 medium before and after culture was determined by measuring the ultraviolet absorption spectrum of the medium before the inoculation and after culture. After culture, the medium was passed through a filter of 0.22 µm, and the ultraviolet absorption spectrum of the filtrate obtained was then measured. The results obtained are shown in Fig. 2. The results shown in Fig. 2 reveals that microorganisms which assimilate a phenolic compound such as o-cresol are present in the intestine of a termite.

### Example 3 (Screening by m-cresol)

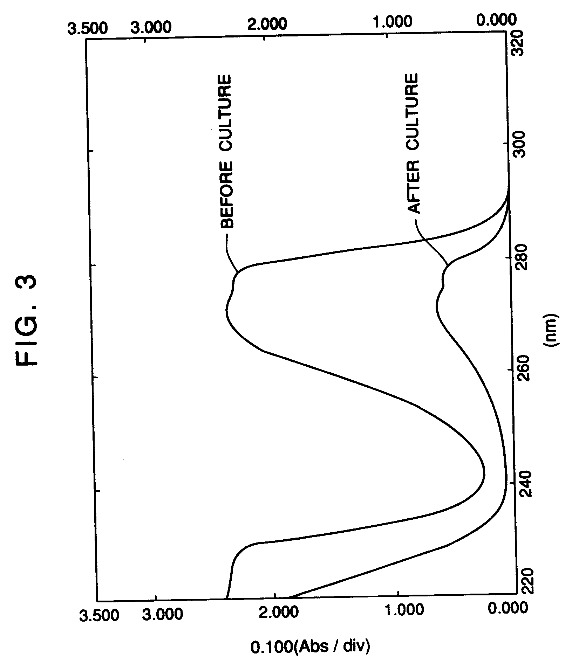

The same operation as that performed in Example 2 were performed except that m-cresol was used in place of o-cresol. As shown in Fig. 3, a reduction in the amount of m-cresol contained in the medium was apparent. This indicates that microorganisms which assimilate m-cresol are present in the intestine of a termite.

### Example 4 (Screening by p-cresol)

The same operation as that performed in Example 2 were performed except that p-cresol was used in place of o-cresol. As shown in Fig. 3, a reduction in the amount of p-cresol contained in the medium was indicated. This shows that microorganisms which assimilate p-cresol are present in the intestine of a termite.

### Example 5 (Acquisition of isolated strain using phenol)

The medium (containing grown bacteria) obtained by culturing the M9 medium (containing 0.05 % phenol and 0.05 % yeast extract) used in Example 1 was coated on the surface of a phenol-containing M9 agar medium (containing 0.05 % phenol and 1.2 % agar), followed by culture under aerobic conditions at 30°C for 2 days. The colonies which grew well on the agar medium were collected to obtain an isolated strain. As a result of examination of the mycological characters of a portion of the isolated strains, the results shown below were obtained. It was concluded that the isolated strain is Pseudomonas cepacia.

### A. Morphological character

(1) Gram's stain: negative (2) Size and form of strain: bacillus having a length of 1.0 to 2.0 µm and a width of about 0.5 µm
(3) Mobility: present

### B. Growth states in various media

**Table 1**

| Medium | Culture Temperature (°C) | Growth state |
|---|---|---|
| Blood agar | 37 | + |
| Lactose agar | 37 | + |
| Chocolate agar | 37 | ++ |
| GMA | 37 | - |
| Scyllo | 37 | - |
| Nutrient agar | 4 | - |
| Nutrient agar | 25 | ± |
| Nutrient agar | 37 | + |
| Nutrient agar | 41 | ± |

### C. Physiological character

(1) Distinction between aerobic and anaerobic: obligate aerobic
(2) Decomposition type of sugar: oxidative
(3) Generation of oxidase: +
(4) Reduction of silver nitrate: +
(5) Generation of hydrogen sulfide: -
(6) Generation of indole: -
(7) Generation of urease: -
(8) Liquefaction of gelatin: -
(9) Hydrolysis of arginine: -
(10) Decarboxylation of lysine: +
(11) Decarboxylation of ornithine: -
(12) Utilization of citric acid: +
(13) Methylcarbinol acetyl reaction (VP reaction) : -
(14) Detection of tryptophan deaminase: -
(15) ONPG: -
(16) Utilization of carbohydrates:
   - Grape sugar:: +
   - Fractose:: +
   - Maltose:: +
   - Galactose:: +
   - Xylose:: +
   - Mannitol:: +
   - Saccharose:: -
   - Lactose:: +
   - Escurin:: -
   - Inositol:: -
   - Sorbitol:: -
   - Rhamnose:: -
   - Melibiose:: -
   - Amygdalin:: -
   - L-(+)-arabinose:: +

This strain was cultured in the M9 medium (5 ml) containing 0.05 % phenol and 0.05 % yeast extract at 30°C. After a predetermined number of days have passed, the bacteria were separated from the medium by passing the medium through a filter of 0.22 µm. The concentration of phenol of the filtrate obtained was determined by measuring light absorption at about 270 nm using a spectrophotometer. The daily changes in the rate of removal (residual rate) of phenol from the medium were measured in the same way as that in Example

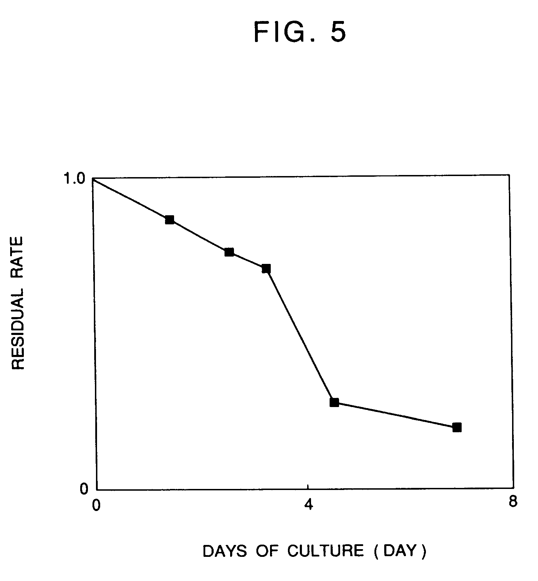

### 1. The results obtained are shown in Fig. 5.

As is obvious from the results shown in Fig. 5, the strain has excellent ability to decompose phenol. Since strains having the ability to decompose phenol are not found in the generally known strains of Pseudomonas cepacia, it was recognized that the strain is a new strain. The strain was named KK01 strain and deposited in Fermentation Research Institute of the Agency of Industrial Science and Technology (Deposition Date: March 11, 1992; Deposition No. FERM P-12869).

### Example 6 (Acquisition of isolated strain using o-cresol)

The medium (containing grown bacteria) obtained by culture in the M9 medium containing 0.02 % o-cresol and 0.05 % yeast extract in Example 2 was coated on the surface of a o-cresol-containing M9 agar medium (containing 0.02 % o-cresol and 1.2 % agar), followed by culture under as aerobic conditions at 30°C for 5 days. The colonies which grew well on the agar medium were collected as an isolated strain. Examination of the mycological characters of a portion of the strain showed the same results as those of the KK01 strain obtained in Example 5. The isolated strain was identified as the KK01 strain.

### Example 7 (Acquisition of isolated strain using m-cresol)

The medium (containing grown bacteria) obtained by culture in the M9 medium containing 0.02 % m-cresol and 0.05 % yeast extract in Example 3 was coated on the surface of a m-cresol-containing M9 agar medium (containing 0.02 % m-cresol and 1.2 % agar), followed by culture under aerobic conditions at 30°C for 5 days. The colonies grown well on the agar medium were collected to obtain an isolated strain. Examination of the mycological characters of a portion of the strain showed the same results as those of the KK01 strain obtained in Example 5. The isolated strain was thus identified as the KK01 strain.

### Example 8 (Acquisition of isolated strain using p-cresol)

The medium (containing grown bacteria) obtained by culture in the M9 medium containing 0.02 % p-cresol and 0.05 % yeast extract in Example 4 was coated on the surface of a p-cresol-containing M9 agar medium (containing 0.02 % p-cresol and 1.2 % agar), followed by culture under aerobic conditions at 30°C for 5 days. The colonies grown well on the agar medium were collected to obtain an isolated strain. Examination of the mycological characters of a portion of the strain showed the same results as those of the KK01 strain obtained in Example 5. The isolated strain was thus identified as the KK01 strain.

### Example 9 (Measurement of removal rate of cresol)

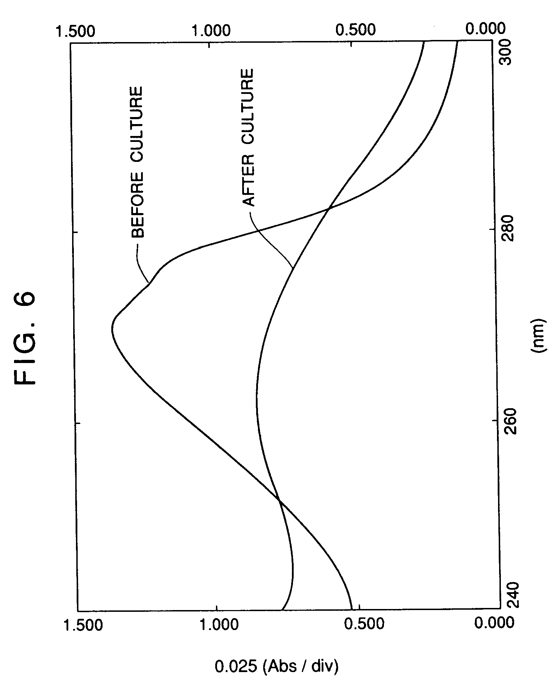

The KK01 strain obtained in Example 6 was cultured in a M9 medium (5 ml) containing 0.02 % o-cresol and 0.05 % yeast extract at 30°C for 7 days. A difference (removal rate) in the amounts of o-cresol contained in the medium before and after culture was determined by measuring the ultraviolet absorption spectrum of the medium before inoculation and after culture. In order to perform the measurement, the medium was passed through a filter of 0.22 µm after culture, and the ultraviolet absorption spectrum of the filtrate obtained was measured. The results obtained are shown in Fig. 6.

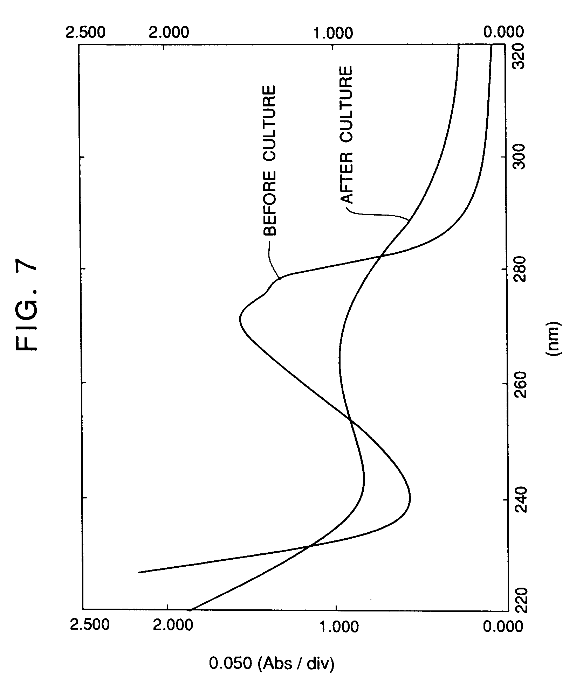

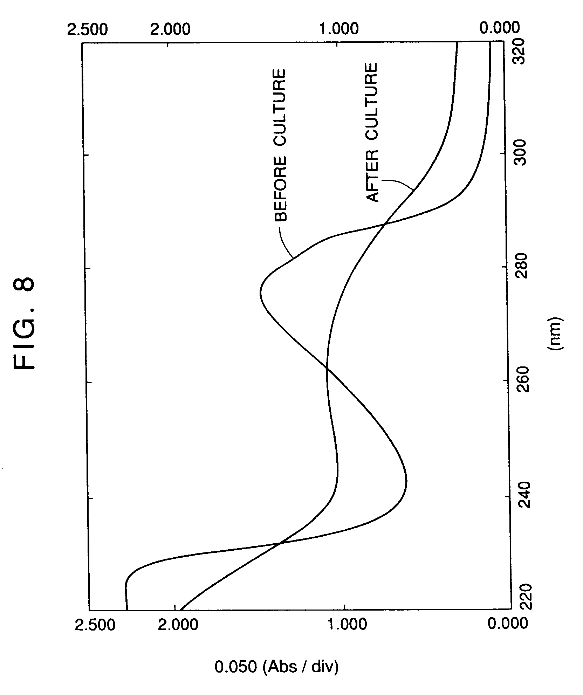

The removal rates of m-cresol and p-cresol were measured by the same method as that described above except that o-cresol was replaced by m-cresol and p-cresol, respectively. The results obtained are shown in Figs. 7 and 8. As is obvious from the results shown in Figs. 6 to 8, the strain of the present invention has not only the ability to decompose phenol but also the ability to decompose cresol. This property is not exhibited by generally known strains of Psuedomonas cepacia.

### Example 10

The KK01 strain was inoculated into the synthetic waste water artificially formed having the composition below, and was cultured under aerobic conditions at 30°C. The changes with time of the amount of phenol contained in the medium were determined by measuring the ultraviolet absorption spectrum of the medium in the same manner as that employed in each of the examples.

### Synthetic waste water composition:

| | |
|---|---|
| Phenol | 200 mg |
| o-cresol | 50 mg |
| m-cresol | 40 mg |
| p-cresol | 50 mg |
| NH₄Cl | 200 mg |
| KH₂PO₄ | 272 mg |
| Na₂HPO₄ | 284 mg |
| Water | 1 l |
| pH (7.0) | |

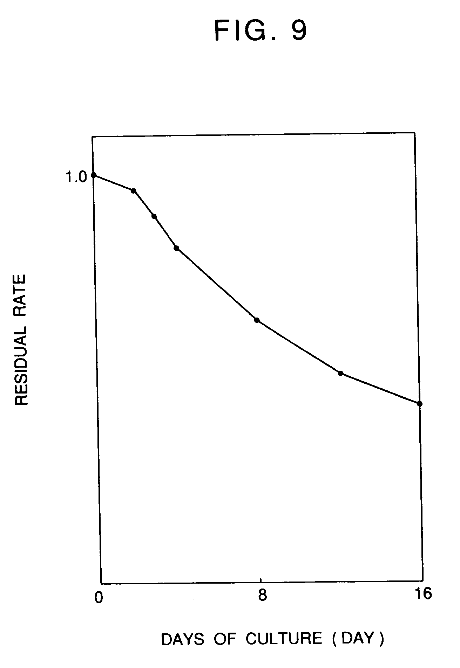

The results obtained are shown in Fig. 9. It was determined from the results that the KK01 strain has the ability to decompose the phenol and cresol contained in the synthetic waste water.

### Example 11 (Screening by furfural)

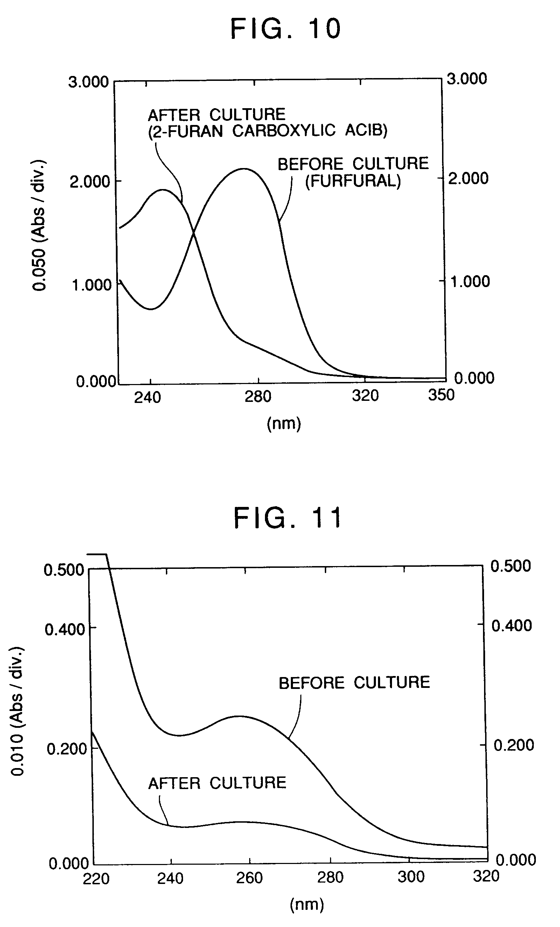

Ten worker termites Nasutitermes takasagoensis were placed on a plate, and ethyl alcohol (95 %) was poured into the plate so as to sterilize the surfaces of the termites. The ethyl alcohol was removed by washing the termites twice in the M9 medium containing 0.02 % furfural. After washing, the intestine was picked out from each termite with a pair of tweezers, and was then ground in the M9 medium containing 0.02 % furfural to obtain a liquid mixture containing the ground intestines. A part of the resulting mixture was inoculated into the M9 medium containing 0.02 % furfural and 0.05 % yeast extract, followed by culture under aerobic conditions at 30°C for 15 days. The absorbance (230 to 350 nm) of the medium was measured before and after culture by a spectrophotometer. In order to perform the measurement, the medium was filtered through a filter of 0.22 µm for removing the bacteria and the like after culture and was then measured. The results obtained are shown in Fig. 10. As shown in Fig. 10, an absorption peak at about 280 nm which indicates the presence of furfural appears in the spectrum measured before culture and disappears from the spectrum measured after culture. While a peak indicating the presence of 2-furan carboxylic acid appears at about 247 nm. The results show that furfural is converted to 2-furan carboxylic acid by culture of the isolated bacteria.

### Example 12 (Acquisition of isolated strain using furfural)

The medium (containing grown bacteria) obtained by culture in the M9 medium (further containing 0.02 % furfural and 0.05 % yeast extract) in Example 11 was coated on the surface of a furfural-containing M9 medium (containing 0.05 % furfural and 1.2 % agar), followed by culture at 30°C. The colonies which grew well on the medium were collected to obtain an isolated strain. Examination of the mycological character of a portion of the isolated stain showed the same results as those of the KK01 strain obtained in Example 5. The isolated strain was thus identified as the KK01 strain.

### Example 13 (Screening by tetrahydrofuran)

Microorganisms having the ability to decompose tetrahydrofuran were isolated from the intestines of termites by the same method as that employed in Example 11 except that tetrahydrofuran was used in place of furfural. The absorbance (220 to 320 nm) of the medium was measured before and after culture. The results obtained are shown in Fig. 11. It was confirmed by the results shown in Fig. 11 that microorganisms having the ability to decompose tetrahydrofuran are present in the intestine of a termite.

### Example 14 (Acquisition of isolated strain using tetrahydrofuran)

The medium (containing grown bacteria) obtained by culture in the M9 medium (further containing 0.02 % tetrahydrofuran and 0.05 % yeast extract) in Example 13 was coated on the surface of a tetrahydrofuran-containing M9 medium (containing 0.05 % tetrahydrofuran and 1.2 % agar), followed by culture at 30°C. The colonies which grew well on the medium were collected to obtain an isolated strain. Examination of the mycological characters of a portion of the isolated strain showed the same results as those of the KK01 strain obtained in Example 5. The isolated strain was thus identified as the KK01 strain.

### Example 15 (Screening by furfuryl alcohol)

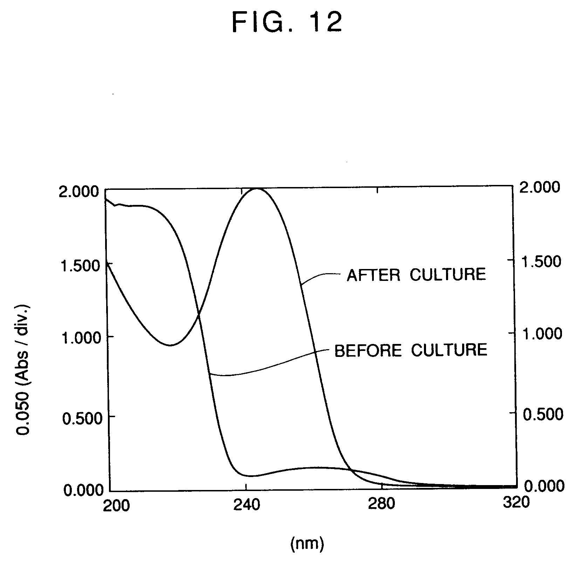

Microorganisms having the ability to decompose furfuryl alcohol were isolated from the intestines of termites by the same method as that employed in Example 11 except that furfuryl alcohol was used in place of furfural. The absorbance (220 to 320 nm) of the medium was measured before and after culture. The results obtained are shown in Fig. 12. It was confirmed by the results shown in Fig. 12 that microorganisms having the ability to decompose furfuryl alcohol are present in the intestine of a termite.

### Example 16 (Acquisition of isolated strain using furfuryl alcohol)

The medium (containing grown bacteria) obtained by culture in the M9 medium (further containing 0.02 % furfuryl alcohol and 0.05 % yeast extract) in Example 15 was coated on the surface of a tetrahydrofuran-containing M9 medium (containing 0.05 % furfural alcohol and 1.2 % agar), followed by culture at 30°C. The colonies which grew well on the medium were collected to obtain an isolated strain. Examination of the mycological character of a portion of the isolated strain showed the same results as those of the KK01 strain obtained in Example 5. The isolated strain was thus identified as the KK01 strain.

### Example 17 (Screening by cumaran)

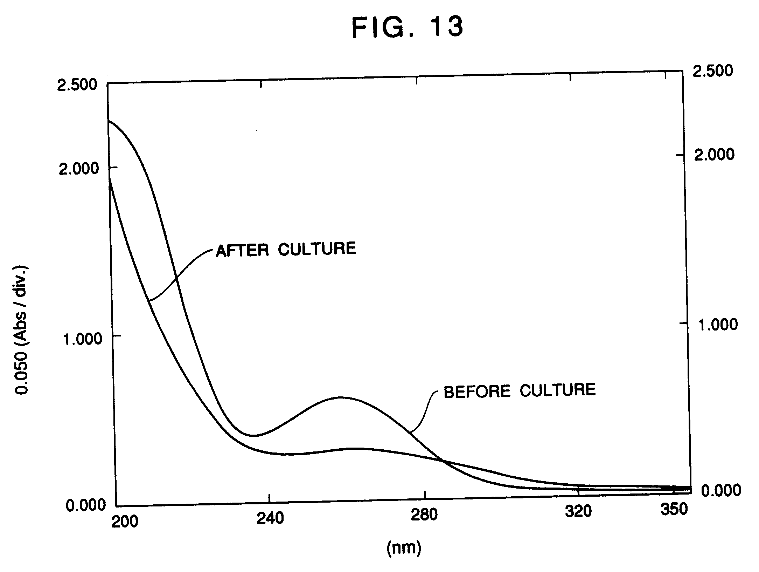

Microorganisms having the ability to decompose cumaran were isolated from the intestines of termites by the same method as that employed in Example 11 except that cumaran was used in place of furfural. The absorbance (200 to 350 nm) of the medium was measured before and after culture. The results obtained are shown in Fig. 13. It was confirmed by the results shown in Fig. 13 that microorganisms having the ability to decompose cumaran are present in the intestine of a termite.

### Example 18 (Acquisition of isolated strain using cumaran)

The medium (containing grown bacteria) obtained by culture in the M9 medium (further containing 0.02 % cumaran and 0.05 % yeast extract) in Example 15 was coated on the surface of a tetrahydrofuran-containing M9 medium (containing 0.05 % cumaran and 1.2 % agar), followed by culture under aerobic conditions at 30°C. The colonies grown well on the medium were collected to obtain an isolated strain. Examination of the mycological characters of a portion of the isolated strain showed the same results as those of the KK01 strain obtained in Example 5. The isolated strain was thus identified as the KK01 strain.

### Example 19 (Generation of 2-furan carboxylic acid)

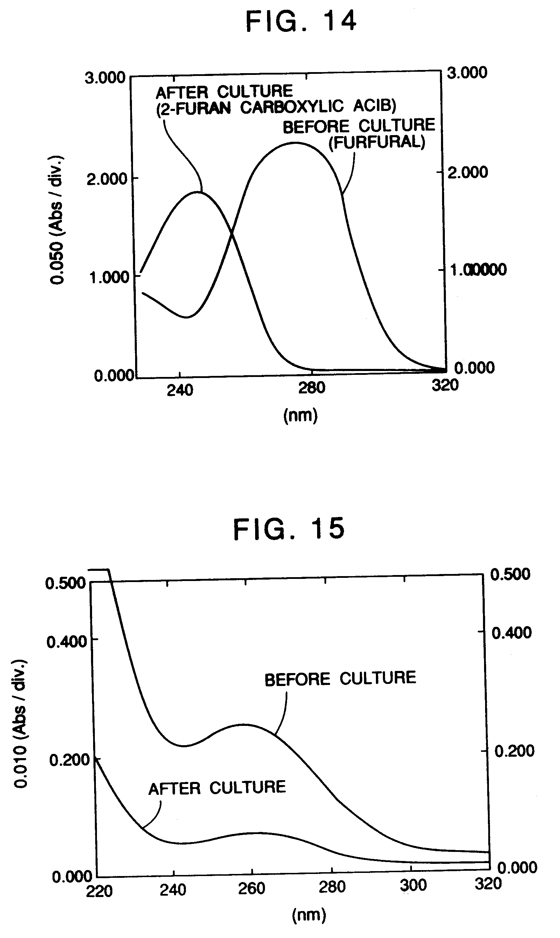

The KK01 strain obtained in Example 12 was cultured in a M9 medium (5 ml) containing 0.05 % furfural ad 0.05 % yeast extract at 30°C for 10 days. The absorbance (230 to 320 nm) of the medium was measured before and after culture. In order to perform the measurement, the medium was filtered through a filter of 0.22 µm after culture and the filtrate was then measured. The results obtained are shown in Fig. 14. As is obvious from the results shown in Fig. 14, furfural is converted to 2-furan carboxylic acid by culture of the KK01 strain.

### Example 20 (Decomposition of tetrahydrofuran)

The KK01 strain was cultured by the same method as that employed in Example 19 except that tetrahydrofuran was used in place of furfural. The absorbance (220 to 320 nm) of the medium was measured. The results obtained are shown in Fig. 15. As is obvious from the results shown i Fig. 15, tetrahydrofuran is decomposed by a culture of the KK01 strain.

### Example 21 (Decomposition of furfural alcohol)

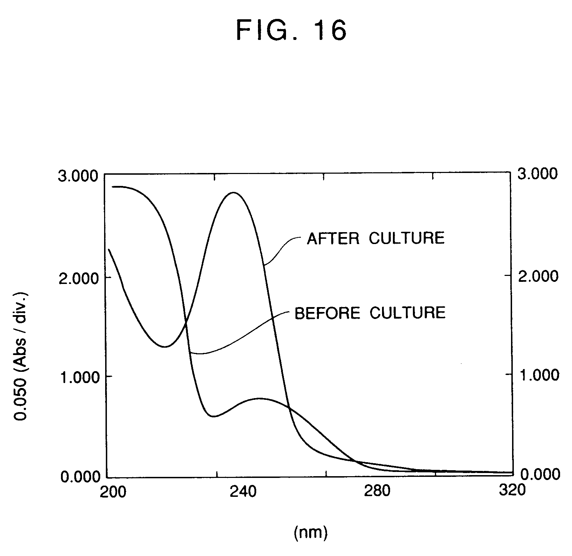

The KK01 strain was cultured by the same method as that employed in Example 19 except that furfuryl alcohol was used in place of furfural. The absorbance (200 to 350 nm) of the medium was measured. The results obtained are shown in Fig. 16. As is obvious from the results shown i Fig. 16, furfuryl alcohol is decomposed by a culture of the KK01 strain.

### Example 22 (Decomposition of cumaran)

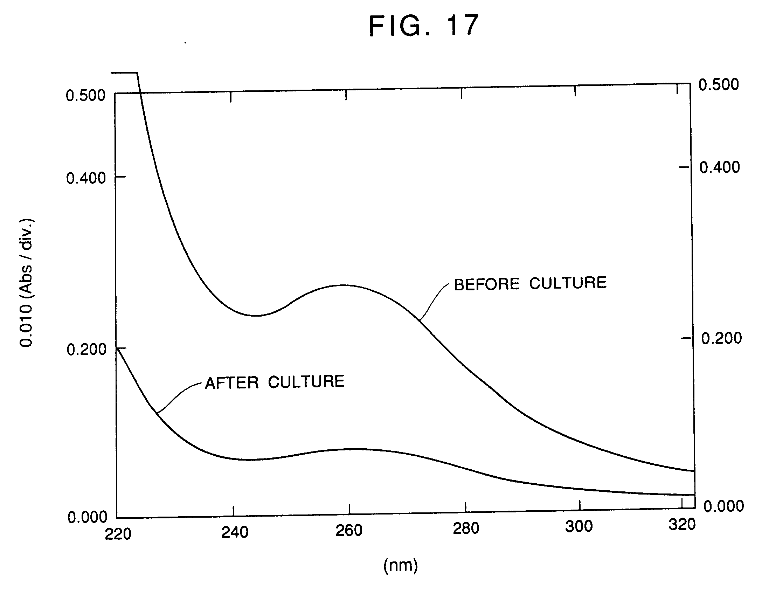

The KK01 strain was cultured by the same method as that employed in Example 19 except that cumaran was used in place of furfural. The absorbance (220 to 320 nm) of the medium was measured. The results obtained are shown in Fig. 17. As is obvious from the results shown in Fig. 17, cumaran is decomposed by a culture of the KK01 strain.

As seen from the above results, the strain obtained in the present invention has the ability to decompose the furan compounds, and this property is not observed in generally known strains of Pseudomonas cepacia.

The KK01 strain can be cultured in a medium which is generally used for bacteria Pseudomonas. Although the strain can be grown adequately in a medium containing as a single carbon source phenol, cresol or a furan compound, glucose can also appropriately be used. For example, peptone can be used as a nitrogen source singly or in combination with other substances. The yeast extract, potassium primary phosphate, ammonium chloride and the like may be added according to demand. The culture can be made under aerobic conditions, and either liquid culture or solid culture may be made. The culture temperature is preferably 30°C.

### Example 23

A synthetic waste water having the composition below was synthesized, and the KK01 strain was inoculated into the waste water, followed by culture under aerobic conditions at 30°C. The changes with time of the amount of the furan compound contained in the medium were determined by measuring the ultraviolet absorption of the medium. A sample of the medium was filtered by through filter of 0.22 µm and the filtrate was then measured.

### Synthetic waste water composition:

| | |
|---|---|
| Tetrahydrofuran | 50 mg |
| Furfural | 50 mg |
| Furfuryl alcohol | 50 mg |
| Cumaran | 50 mg |
| NH₄Cl | 200 mg |
| KH₂PO₄ | 272 mg |
| Na₂HPO₄ | 284 mg |
| Water | 1 l |
| pH (7.0) | |

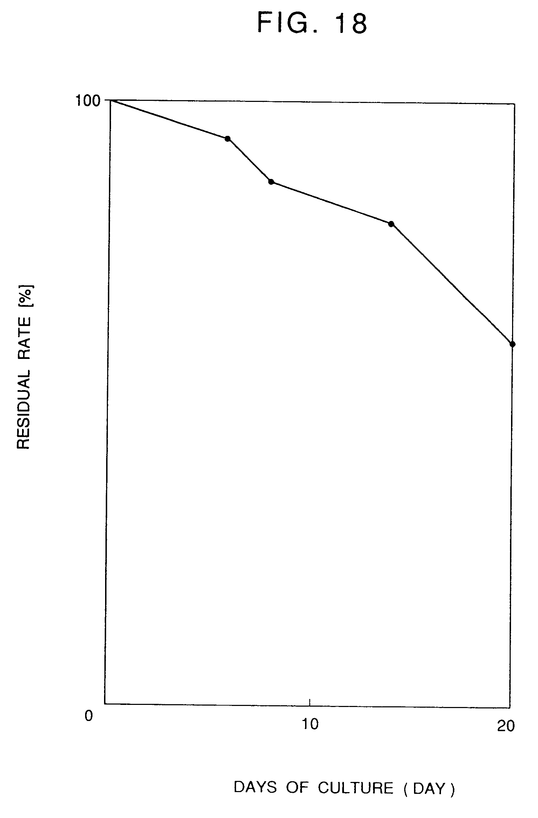

The results obtained are shown in Fig. 18. The results shown in Fig. 18 reveal that the KK01 strain has the ability to decompose the furan compounds contained in the synthetic waste water.

The present invention establishes a method of obtaining microorganisms from the intestine of a termite having the ability to decompose phenolic compounds or furan compounds. The microorganisms obtained were suitable for biologically decomposing the phenolic compounds or furan compounds contained in the waste water.

The use of the microorganisms obtained by the above described method permits efficient biological treatment of waste water containing phenolic compounds or furan compounds which are not easily decomposed under natural conditions. This is particularly advantageous since microorganisms having the ability to decompose cresol, tetrahydrofuran and cumaran have to-date not been reported. The present invention enables biological decomposition of these compounds contained in waste water. The present invention also enables biological decomposition of various chemical substances such as phenolic compounds, furan compounds and the like using a single strain.

A method of biologically decomposing a phenolic compound or a furan compound has the step of decomposing a phenolic compound or a furan compound by bringing an aqueous solution containing the phenolic compound or furan compound into contact with microorganisms derived from the intestine of a termite and having the ability to decompose the phenolic compound or furan compound. The microorganisms having the ability to decompose the phenolic compound or the furan compound is obtained by culturing the microorganisms isolated from the intestine of a termite in a medium containing as a single carbon source the phenolic compound or the furan compound, and recovering the microorganisms grown.

## Claims

1. A method of biologically decomposing a phenolic compound comprising the step of:
deriving microorganisms from the intestine of a termite which can decompose a phenolic compound; and
decomposing said phenolic compound by bringing an aqueous solution containing said phenolic compound into contact with said microorganisms.

2. A method of biologically decomposing a phenolic compound according to Claim 1, wherein said termite is Nasutitermes takasagoensis.

3. A method of biologically decomposing a phenolic compound according to Claim 1, wherein said microorganisms are bacteria.

4. A method of biologically decomposing a phenolic compound according to Claim 3, wherein said bacteria are Pseudomonas cepacia.

5. A method of biologically decomposing a phenolic compound according to Claim 1, wherein said phenolic compound is at least one selected from the group consisting of phenol, o-cresol, p-cresol and m-cresol.

6. A method of obtaining microorganisms which can decompose a phenolic compound comprising the steps of:
isolating the microorganisms which can decompose a phenolic compound obtained from the intestine of a termite;
culturing said microorganisms in a medium containing said phenolic compound as a single carbon source, and
recovering said microorganisms which have grown on the culture.

7. A method of obtaining microorganisms which can decompose a phenolic compound according to Claim 6, wherein said termite is Nasutitermes takasagoensis.

8. A method of obtaining microorganisms which can decompose a phenolic compound according to Claim 6, wherein said medium is suitable for culturing bacteria.

9. A method of obtaining microorganisms which can decompose a phenolic compound according to Claim 6, wherein said phenolic compound is at least one selected from the group consisting of phenol, o-cresol, p-cresol and m-cresol.

10. A method of biologically decomposing a furan compound comprising the step of:
deriving microorganisms from the intestine of a termite which can decompose a furan compound; and
decomposing said furan compound by bringing an aqueous solution containing said furan compound into contact with said microorganisms.

11. A method of biologically decomposing a furan compound according to Claim 10, wherein said termite is Nasutitermes takasagoensis.

12. A method of biologically decomposing a furan compound according to Claim 10, wherein said microorganisms are bacteria.

13. A method of biologically decomposing a furan compound according to Claim 12. wherein said bacteria are Pseudomonas cepacia.

14. A method of biologically decomposing a furan compound according to Claim 10, wherein said furan compound is at least one selected from the group consisting of furfural, tetrahydrofuran, furfuryl alcohol and cumaran.

15. A method of obtaining microorganisms which can decompose a furan compound comprising the steps of:
isolating said microorganisms which can decompose a furan compound from the intestine of a termite; and
culturing said microorganisms in a medium containing a furan compound as a single carbon source, and;
recovering the microorganisms which have grown on the culture.

16. A method of obtaining microorganisms which can decompose a furan compound according to Claim 15, wherein said termite is Nasutitermes takasagoensis.

17. A method of obtaining microorganisms which can decompose a furan compound according to Claim 15, wherein said medium is suitable for culturing bacteria.

18. A method of obtaining microorganisms which can decompose a furan compound according to Claim 15, wherein said furan compound is at least one selected from the group consisting of furfural, tetrahydrofuran, furfuryl alcohol and cumaran.

19. A method of producing 2-furan carboxylic acid from furfural comprising the step of:
deriving microorganisms from the intestine of a termite; and
contacting said microorganisms with an aqueous solution containing furfural to oxidize said furfural to 2-furan carboxylic acid.

20. A method of producing 2-furan carboxylic acid according to claim 19, wherein said termite is Nasutitermes takasagoensis.

21. A method of producing 2-furan carboxylic acid according to claim 19, wherein said microorganisms are bacteria.

22. A method of producing 2-furan carboxylic acid according to claim 21, wherein said bacteria are Pseudomonas cepacia.

23. A method of producing 2-furan carboxylic acid according to claim 22, wherein said bacteria are Pseudomonas cepacia KK01 (Ferm BP4235).

24. A method of biologically decomposing a phenolic compound according to Claim 4, wherein said bacteria are Pseudomonas cepacia KKO1 (Ferm BP4235).

25. A method of biologically decomposing a furan compound according to Claim 13, wherein said bacteria are Pseudomonas cepacia KKO1 (Ferm BP4235).

26. An isolate Pseudomonas cepacia KKO1(Ferm BP4235) derived from the intestine of a termite which can decompose a phenolic compound and a furan compound.

27. A process for decomposing organic compounds with microorganism, comprising the step of bringing the microorganism into contact with the compounds wherein the organic compounds are phenolic and furan compounds,
**characterized in that**
the microorganism is Pseudomonas cepacia KK01 (FERM BP-4235).

## Patentansprüche

1. Verfahren zum biologischen Abbau einer Phenolverbindung, das die nachstehenden Schritte umfaßt:
Gewinnung von Mikroorganismen aus dem Darm einer Termite, die eine Phenolverbindung abbauen können; und
Abbau der Phenolverbindung durch In-Kontakt-Bringen einer wäßrigen Lösung, die die Phenolverbindung enthält, mit den Mikroorganismen.

2. Verfahren zum biologischen Abbau einer Phenolverbindung nach Anspruch 1, in dem es sich bei der Termite um Nasutitermes takasagoensis handelt.

3. Verfahren zum biologischen Abbau einer Phenolverbindung nach Anspruch 1, in dem es sich bei den Mikroorganismen um Bakterien handelt.

4. Verfahren zum biologischen Abbau einer Phenolverbindung nach Anspruch 3, in dem es sich bei den Bakterien um Pseudomonas cepacia handelt.

5. Verfahren zum biologischen Abbau einer Phenolverbindung nach Anspruch 1, in dem die Phenolverbindung mindestens eine Verbindung ist, die aus der Gruppe ausgewählt ist, die aus Phenol, o-Kresol, p-Kresol und m-Kresol besteht.

6. Verfahren zur Gewinnung von Mikroorganismen, die eine Phenolverbindung abbauen können, das die nachstehenden Schritte umfaßt:
Isolierung der Mikroorganismen, die eine Phenolverbindung abbauen können und aus dem Darm einer Termite erhalten wurden;
Züchtung der Mikroorganismen in einem Medium, das die Phenolverbindung als einzige Kohlenstoffquelle enthält, und
Gewinnung der Mikroorganismen, die in der Kultur gezüchtet wurden.

7. Verfahren zur Gewinnung von Mikroorganismen, die eine Phenolverbindung abbauen können, nach Anspruch 6, in dem es sich bei der Termite um Nasutitermes takasagoensis handelt.

8. Verfahren zur Gewinnung von Mikroorganismen, die eine Phenolverbindung abbauen können, nach Anspruch 6, wobei das Medium zum Züchten von Bakterien geeignet ist.

9. Verfahren zur Gewinnung von Mikroorganismen, die eine Phenolverbindung abbauen können, nach Anspruch 6, wobei die Phenolverbindung mindestens eine Verbindung ist, die aus der Gruppe ausgewählt ist, die aus Phenol, o-Kresol, p-Kresol und m-Kresol besteht.

10. Verfahren zum biologischen Abbau einer Furanverbindung, das die nachstehenden Schritte umfaßt:
Gewinnung von Mikroorganismen aus dem Darm einer Termite, die eine Furanverbindung abbauen können; und
Abbau der Furanverbindung durch In-Kontakt-Bringen einer wäßrigen Lösung, die die Furanverbindung enthält, mit den Mikroorganismen.

11. Verfahren zum biologischen Abbau einer Furanverbindung nach Anspruch 10, wobei es sich bei der Termite um Nasutitermes takasagoensis handelt.

12. Verfahren zum biologischen Abbau einer Furanverbindung nach Anspruch 10, in dem es sich bei den Mikroorganismen um Bakterien handelt.

13. Verfahren zum biologischen Abbau einer Furanverbindung nach Anspruch 12, in dem es sich bei den Bakterien um Pseudomonas cepacia handelt.

14. Verfahren zum biologischen Abbau einer Furanverbindung nach Anspruch 10, in dem die Furanverbindung mindestens eine Verbindung ist, die aus der Gruppe ausgewählt ist, die aus Furfural, Tetrahydrofuran, Furfurylalkohol und Cumaran besteht.

15. Verfahren zur Gewinnung von Mikroorganismen, die eine Furanverbindung abbauen können, das die nachstehenden Schritte umfaßt:
Isolierung der Mikroorganismen, die eine Furanverbindung abbauen können, aus dem Darm einer Termite; und
Züchtung der Mikroorganismen in einem Medium, das eine Furanverbindung als einzige Kohlenstoffquelle enthält; und
Gewinnung der Mikroorganismen, die in der Kultur gezüchtet wurden.

16. Verfahren zur Gewinnung von Mikroorganismen, die eine Furanverbindung abbauen können, nach Anspruch 15, in dem es sich bei der Termite um Nasutitermes takasagoensis handelt.

17. Verfahren zur Gewinnung von Mikroorganismen, die eine Phenolverbindung abbauen können, nach Anspruch 15, wobei das Medium zum Züchten von Bakterien geeignet ist.

18. Verfahren zur Gewinnung von Mikroorganismen, die eine Furanverbindung abbauen können, nach Anspruch 15, wobei die Phenolverbindung mindestens eine Verbindung ist, die aus der Gruppe ausgewählt ist, die aus Furfural, Tetrahydrofuran, Furfurylalkohol und Cumaran besteht.

19. Verfahren zur Herstellung von 2-Furancarbonsäure aus Furfural, das die nachstehenden Schritte umfaßt:
Gewinnung von Mikroorganismen aus dem Darm einer Termite; und
In-Kontakt-Bringen der Mikroorganismen mit einer wäßrigen Lösung, die Furfural enthält, um das Furfural zu 2-Furancarbonsäure zu oxidieren.

20. Verfahren zur Herstellung von 2-Furancarbonsäure nach Anspruch 19, in dem es sich bei der Termite um Nasutitermes takasagoensis handelt.

21. Verfahren zur Herstellung von 2-Furancarbonsäure nach Anspruch 19, in dem es sich bei den Mikroorganismen um Bakterien handelt.

22. Verfahren zur Herstellung von 2-Furancarbonsäure nach Anspruch 21, in dem es sich bei den Bakterien um Pseudomonas cepacia handelt.

23. Verfahren zur Herstellung von 2-Furancarbonsäure nach Anspruch 22, in dem es sich bei den Bakterien um Pseudomonas cepacia KK01 (Ferm BP4235) handelt.

24. Verfahren zum biologischen Abbau einer Phenolverbindung nach Anspruch 4, in dem es sich bei den Bakterien um Pseudomonas cepacia KK01 (Ferm BP4235) handelt.

25. Verfahren zum biologischen Abbau einer Furanverbindung nach Anspruch 13, in dem es sich bei den Bakterien um Pseudomonas cepacia KK01 (Ferm BP4235) handelt.

26. Isoliertes Pseudomonas cepacia KK01 (Ferm BP4235), das aus dem Darm einer Termite gewonnen wurde, das eine Phenolverbindung und eine Furanverbindung abbauen kann.

27. Verfahren zum Abbau organischer Verbindungen mit Mikroorganismen, das den Schritt des In-Kontakt-Bringens des Mikroorganismus mit den Verbindungen umfaßt, wobei es sich bei den organischen Verbindungen um Phenol- und Furanverbindungen handelt,
**dadurch gekennzeichnet, daß**
es sich bei dem Mikroorganismus um Pseudomonas cepacia KK01 (Ferm BP-4235) handelt.

## Revendications

1. Procédé de décomposition biologique d'un composé phénolique, comprenant les étapes consistant :
à obtenir des micro-organismes pouvant décomposer un composé phénolique à partir de l'intestin d'un termite ; et
à décomposer ledit composé phénolique en mettant une solution aqueuse contenant ledit composé phénolique en contact avec lesdits micro-organismes.

2. Procédé de décomposition biologique d'un composé phénolique suivant la revendication 1, dans lequel le termite est Nasutitermes takasagoensis.

3. Procédé de décomposition biologique d'un composé phénolique suivant la revendication 1, dans lequel les micro-organismes sont des bactéries.

4. Procédé de décomposition biologique d'un composé phénolique suivant la revendication 3, dans lequel les bactéries consistent en Pseudomonas cepacia.

5. Procédé de décomposition biologique d'un composé phénolique suivant la revendication 1, dans lequel ledit composé phénolique consiste en au moins un compose phénolique choisi dans le groupe consistant en phénol, o-crésol, p-crésol et m-crésol.

6. Procédé d'obtention de micro-organismes pouvant décomposer un composé phénolique, comprenant les étapes consistant :
à isoler les micro-organismes pouvant décomposer un composé phénolique obtenus à partir de l'intestin d'un termite ;
à cultiver lesdits micro-organismes dans un milieu contenant ledit composé phénolique comme seule source de carbone, et
à recueillir lesdits micro-organismes qui se sont développés dans la culture.

7. Procédé d'obtention de micro-organismes pour décomposer un composé phénolique suivant la revendication 6, dans lequel le termite consiste en Nasutitermes takasagoensis.

8. Procédé d'obtention de micro-organismes pouvant décomposer un composé phénolique suivant la revendication 6, dans lequel le milieu convient à la culture de bactéries.

9. Procédé d'obtention de micro-organismes pouvant décomposer un composé phénolique suivant la revendication 6, dans lequel le composé phénolique consiste en au moins un composé choisi dans le groupe consistant en phénol, o-crésol, p-crésol et m-crésol.

10. Procédé de décomposition biologique d'un dérivé de furanne, comprenant les étapes consistant :
à obtenir à partir de l'intestin d'un termite des micro-organismes pouvant décomposer un dérivé de furanne ; et
à décomposer ledit dérivé de furanne en mettant une solution aqueuse contenant ledit dérivé de furanne en contact avec lesdits micro-organismes.

11. Procédé de décomposition biologique d'un dérivé de furanne suivant la revendication 10, dans lequel le termite consiste en Nasutitermes takasagoensis.

12. Procédé de décomposition biologique d'un dérivé de furanne suivant la revendication 10, dans lequel les micro-organismes sont des bactéries.

13. Procédé de décomposition biologique d'un dérivé de furanne suivant la revendication 12, dans lequel les bactéries consistent en Pseudomonas cepacia.

14. Procédé de décomposition biologique d'un dérivé de furanne suivant la revendication 10, dans lequel ledit dérivé de furanne consiste en au moins un composé choisi dans le groupe consistant en le furfural, le tétrahydrofuranne, l'alcool furfurylique et le coumaranne.

15. Procédé d'obtention de micro-organismes pouvant décomposer un dérivé de furanne, comprenant les étapes consistant :
à isoler lesdits micro-organismes pouvant décomposer le dérivé de furanne à partir de l'intestin d'un termite ;
à cultiver lesdits micro-organismes dans un milieu contenant un dérivé de furanne comme seule source de carbone, et
à recueillir les micro-organismes qui se sont développés sur la culture.

16. Procédé d'obtention de micro-organismes pouvant décomposer un dérivé de furanne suivant la revendication 15, dans lequel le termite consiste en Nasutitermes takasagoensis.

17. Procédé d'obtention de micro-organismes pouvant décomposer un dérivé de furanne suivant la revendication 15, dans lequel le milieu convient à la culture de bactéries.

18. Procédé d'obtention de micro-organismes pouvant décomposer un dérivé de furanne suivant la revendication 15, dans lequel ledit dérivé de furanne consiste en au moins un composé choisi dans le groupe consistant en le furfural, le tétrahydrofuranne, l'alcool funfurylique et le coumaranne.

19. Procédé de production d'acide 2-furannecarboxylique à partir de furfural, comprenant les étapes consistant :
à obtenir des micro-organismes à partir de l'intestin d'un termite ; et
à mettre en contact lesdits micro-organismes avec une solution aqueuse contenant du funfural pour oxyder ce furfural en acide 2-furanne-carboxylique.

20. Procédé de production d'acide 2-furanne-carboxylique suivant la revendication 19, dans lequel le termite consiste en Nasutitermes takasagoensis.

21. Procédé de production d'acide 2-furanne-carboxylique suivant la revendication 19, dans lequel les micro-organismes consistent en bactéries.

22. Procédé de production d'acide 2-furanne-carboxylique suivant la revendication 21, dans lequel les bactéries consistent en Pseudomonas cepacia.

23. Procédé de production d'acide 2-furanne-carboxylique suivant la revendication 22, dans lequel les bactéries consistent en Pseudomonas cepacia KK01 (Ferm BP4235).

24. Procédé de décomposition biologique d'un composé phénolique suivant la revendication 4, dans lequel les bactéries consistent en Pseudomonas cepacia KK01 (Ferm BP4235).

25. Procédé de décomposition biologique d'un dérivé de furanne suivant la revendication 13, dans lequel les bactéries consistent en Pseudomonas cepacia KK01 (Ferm BP4235).

26. Isolat de Pseudomonas cepacia KK01 (Ferm BP4235) dérivé de l'intestin d'un termite qui peut décomposer un composé phénolique et un dérivé de furanne.

27. Procédé pour décomposer des composés organiques avec un micro-organisme, comprenant l'étape consistant à mettre le micro-organisme en contact avec les composés, dans lequel les composés organiques sont des composés phénoliques et des dérivés de furanne,
caractérisé en ce que
le micro-organisme consiste en Pseudomonas cepacia KK01 (FERM BP-4235).
